# EUROPÄISCHE PATENTANMELDUNG

(11) **EP 4 737 539 A2**
(43) Veröffentlichungstag der Anmeldung: **06.05.2026**
(21) Anmeldenummer: 26164867.9
(22) Anmeldetag: 10.09.2019
(51) Int. Cl.: C10M 107/50

(54) **PACKMITTEL MIT GLEITSCHICHT UND VERFAHREN FÜR PHARMAZEUTISCHE UND KOSMETISCHE STOFFE UND ZUBEREITUNG ZU DESSEN HERSTELLUNG**

(30) Priorität: 10.09.2018 DE 102018122001
(62) Teilanmeldung aus: 19196347.9
(71) Anmelder: SCHOTT Pharma AG & Co. KGaA, 55122 Mainz (DE)
(72) Erfinder: MANGOLD, Stephanie, 55288 Schornsheim (DE); SWEECK, Tamara, 55583 Bad Kreuznach (DE)
(74) Vertreter: Blumbach · Zinngrebe Patentanwälte PartG mbB

(57) **Zusammenfassung**

Die Erfindung betrifft eine Zubereitung zum Auftrag auf die Innenseite eines Hohlkörpers zur Herstellung einer Gleitschicht auf einem Packmittel für Pharmazeutika oder kosmetische Produkte. Die flüssige Zubereitung umfasst hierbei folgende Bestandteile:
- ein reaktives Silikon-System zur Ausbildung eines Silikonnetzwerks der Gleitschicht,
- einen Katalysator zur Katalyse der Vernetzungsreaktion des reaktiven Silikon-Systems.
- zumindest ein nicht reaktives Silikonöl,
- und zumindest einen Diluenten,
wobei der Diluent eine siliziumhaltige Verbindung umfasst und wobei der Gehalt des Diluenten in der Zubereitung 45 Gewichtsprozent oder mehr und 95 Gewichtsprozent oder weniger in der Zubereitung beträgt. Weiterhin betrifft die Erfindung ein Packmittel für Pharmazeutika oder kosmetische Produkte, welches einen zylindrischen Hohlkörper (5) umfasst, der innenseitig mit einer Gleitschicht (10) beschichtet ist, wobei die Gleitschicht (10) ein Silikon-Netzwerk aufweist, in welchem ein Silikonöl aufgenommen ist, wobei der Hohlkörper derart ausgestaltet ist, dass ein Stopfen in den Hohlkörper eingeführt werden kann und der Haftreibungswert der Reibung eines in den Hohlkörper eingesetzten Stopfens an der Gleitschicht maximal 20% größer, als der mittlere Gleitreibungswert ist sowie ein Verfahren zur Herstellung eines Packmittels für Pharmazeutika oder kosmetische Produkte.

## Beschreibung

Die vorliegende Anmeldung ist eine Teilanmeldung der Europäischen Patentanmeldung EP 19196347.9, deren Inhalt vollumfänglich auch Gegenstand dieser Teilanmeldung ist.

### Beschreibung

Die Erfindung betrifft allgemein Behälter zur Aufnahme von Pharmazeutika und kosmetischen Zubereitungen. Im Besonderen betrifft die Erfindung derartige Behälter mit einer Gleitschicht, die das Gleiten eines Kolbens, beziehungsweise Stopfens zur Entleerung des Behälters erleichtert.

Kunststoffbasierte Kosmetika- und Pharmapackmittel sind seit mehreren Jahrzehnten Stand der Technik, bringen jedoch auch verschiedene Herausforderungen mit sich. Im Einsatz als Pharmapackmittel für Spritzenanwendungen besteht die größte Herausforderung darin, einen eingesetzten Stopfen mit geringer Kraft zu bewegen. Eine geringe Haft- und Gleitreibung des Stopfens bewirkt, dass der Inhalt vollständig und zügig aus der Spritze herausgedrückt werden kann und Unannehmlichkeiten für den zu behandelnden Patienten, die durch eine ungleichmäßige Bewegung des Stopfens oder sogar ein Stocken des Stopfens entstehen können, auf ein Minimum reduziert werden.

Aus dem Stand der Technik sind vor allem silikonisierte Pharmapackmittel zur Lösung dieser Herausforderung bekannt.

In der US 4 767 414 A wird das Aufbringen von Silikonöl auf der Innenwandung beschrieben, nachdem die Kunststoffinnenoberfläche mit einem Plasma aktiviert wurde. Wünschenswert wäre es jedenfalls, auf den zusätzlichen Schritt der Plasmaaktivierung verzichten zu können. Zudem kann chemisch nicht kovalent gebundenes Silikonöl gegebenenfalls in den Patienten, insbesondere in die menschliche Blutbahn gelangen.

Die EP 0920879 B1 beschreibt ein Gemisch auf Silikonbasis. Die Mischung aus einem reaktiven Silikonöl und einem nicht reaktiven Silikonöl ermöglicht die Bindung der Silikonschicht an das Substrat, sowie eine gute Gleiteigenschaft des Stopfens.

Eine weitere Herausforderung besteht weiterhin darin, die Gleiteigenschaften einer Gleitschicht unter verschiedenen Einwirkungen auf das Packmittel aufrechtzuerhalten.

Der Erfindung liegt daher die Aufgabe zugrunde, einen Behälter für Pharmazeutika und Kosmetika bereitzustellen, der eine gegenüber äußeren Einwirkungen möglichst unempfindliche Gleitschicht aufweist. Diese Aufgabe wird durch den Gegenstand der unabhängigen Ansprüche gelöst. Vorteilhafte Ausgestaltungen und Weiterbildungen sind in den jeweiligen abhängigen Ansprüchen angegeben.

Demgemäß sieht die Erfindung eine Zubereitung zum Auftrag auf die Innenseite eines Hohlkörpers zur Herstellung eines Gleitfilms bzw. einer Gleitschicht auf einem Packmittel für Pharmazeutika oder kosmetische Produkte vor. Die flüssige Zubereitung umfasst
- ein reaktives Silikon-System zur Ausbildung eines Silikonnetzwerks der Gleitschicht,
- einen Katalysator zur Katalyse der Vernetzungsreaktion des reaktiven Silikon-Systems
- zumindest ein nicht reaktives Silikonöl (vzgsw. Polydimethylsiloxan),
- zumindest einen Diluenten, wobei der Diluent eine siliziumhaltige Verbindung umfasst und wobei der Gehalt des Diluenten in der Zubereitung 45 Gewichtsprozent oder mehr und 95 Gewichtsprozent oder weniger in der Zubereitung beträgt. Gemäß einer Ausführungsform beträgt der Gehalt des Diluenten bevorzugt in der Zubereitung mehr als 45 Gewichtsprozent und weniger als 95 Gewichtsprozent.

Unter einem nicht reaktiven Silikonöl wird im Sinne der Erfindung ein Polysiloxan ohne vernetzbare bzw. polymerisierbare Gruppen verstanden. Insbesondere handelt es sich bei den nicht reaktiven Silikonölen um Polysiloxane mit aliphatischen Resten, beispielsweise um Polydimethylsiloxan.
Innen. So wurde für Hexamethyldisiloxan (HMDSO) als Diluent ein Lösemittelanteil von wenigstens 50 Gewichtsprozent (wt%) als besonders günstig beobachtet. Eine Verminderung des Lösemittelanteils führt sukzessive zu weniger ausgehärteten Schichten. Für geringe Lösemittelanteile würde man an sich eine höhere Schichtfestigkeit sowie eine bessere Aushärtung annehmen, da die Einlagerung von Lösungsmittelmolekülen in den Polymerisationsketten verhindert wird. Dies war jedoch nicht der Fall.
Der Diluent bzw. das Lösungsmittel umfasst erfindungsgemäß eine siliziumhaltige Verbindung, vorzugsweise eine siliziumorganische Verbindung. Überraschenderweise hat sich gezeigt, dass der Si-Gehalt des Diluenten eine entscheidende Rolle bei der Schichthaftung und -aushärtung spielt. Als besonders vorteilhaft hat sich hierbei die Verwendung von Siloxanen bzw. Polysiloxanen wie beispielsweise HMDSO herausgestellt. Die entsprechenden Schichten weisen hierbei eine homogene Zusammensetzung sowie eine gute Schichtanhaftung an das Substrat auf. Insbesondere die gute Schichtanhaftung ist insofern überraschend, da die Substrate organischen Kunststoff umfassen und in der Regel keine Si-O-Bindungen aufweisen, welche mit den in der Zubereitung befindlichen Silikonölen kovalente Wechselwirkungen eingehen und so eine erhöhte Schichtanhaftung erzielen könnten. Es kann daher ohne Einschränkung auf diese Hypothese vermutet werden, dass der siliziumhaltige Diluent auch als eine Art Haftvermittler zwischen dem in der Regel siliziumfreien Substrat und den Silikonölen in der Zubereitung fungiert.

Zudem weisen siliziumhaltige Diluenten auf Siloxanbasis wie beispielsweise HMDSO relativ niedrige Oberflächenspannungen auf, was in Hinblick auf die Benetzung der Substratoberfläche vorteilhaft ist. Generell, ohne Beschränkung auf Siloxan-basierte Diluenten, weist der siliziumhaltige Diluent eine Oberflächenspannung von kleiner als 19 mN/m auf.

Darüber hinaus wird vermutet, dass sich die Silizium- bzw. Siloxanfunktionalitäten des Diluenten vorteilhaft auf die Löslichkeit der Silikonöle im Diluenten und somit auch auf die homogene Verteilung der Silikonöle in der Zubereitung auswirkt, was wiederum eine große Homogenität der entsprechenden Beschichtung ermöglicht.

Im Unterschied dazu konnten aus Zubereitungen mit siliziumfreien Lösungsmitteln wie beispielsweise Toluol als Diluent keine homogenen Schichten erhalten werden. Hierbei konnten die Schichteigenschaften auch nicht durch Erhöhung des Diluentengehaltes signifikant verbessert werden.

Bei der erfindungsgemäßen Zubereitung haben sich dagegen noch höhere Anteile des Diluenten in Bezug auf die Schichteigenschaften der entsprechenden Schicht als vorteilhaft herausgestellt. Es ist daher in Weiterbildung vorgesehen, dass der Diluent in der Zubereitung einen Anteil von mindestens 60 Gewichtsprozent, bevorzugt mehr als 70 Gewichtsprozent, besonders bevorzugt mehr als 80 Gewichtsprozent und ganz besonders bevorzugt mehr als 82 Gewichtsprozent beträgt. Der Diluent der Zubereitung kann also ohne weiteres und mit sehr guten Eigenschaften der daraus hergestellten Gleitschicht mehr als 4/5 des Gesamtgewichts einnehmen.

Eine geringe Schichtfestigkeit führt im Allgemeinen zu einem Versagen der Gleitschicht, da diese dann durch den Stopfen bei der Bewegung im Hohlkörper abgelöst wird oder sich sogar ohne äußeren Krafteinfluss von der Innenwandung ablösen kann. Die gewünschten Werte der Haft- und Gleitreibung ("HGR-Werte") können dann im Allgemeinen nicht erreicht werden. Als Beispiel zeigt sich bei einem zu geringen Gehalt von HMDSO als Diluent, dass die Polymerisation der Reaktionslösung so weit reduziert wird, dass die Reaktionslösung an aufrechtstehenden Proben im Hohlkörper nach unten fließt und sich auf der Unterlage ansammelt.

Hierbei ist der vorteilhafte Einfluss eines hohen Gehaltes an Diluenten bzw. Lösungsmittel in der Zubereitung auf die Schichtfestigkeit der vernetzten Schicht höchst überraschend. So führen geringe Konzentrationen der reaktiven Gruppen in der Regel zu geringeren Polymerisationsgraden und somit zu schwach vernetzten Schichten. Bei der Vernetzung der erfindungsgemäßen Zubereitung werden überraschenderweise jedoch trotz hoher Lösungsmittelanteile bzw. Anteile an Diluent in der Zubereitung Schichten mit einem hohen Vernetzungsgrad und hohen Schichtfestigkeiten erhalten.

Weiterhin kann beobachtet werden, dass die Benetzung durch Zubereitungen mit hohen Anteilen an Diluenten sehr homogen ist. So treten bei den erfindungsgemäßen Diluentengehalten keine Inselbildungen, beispielsweise in Form eines sog. Lotus-Effektes, der Zubereitung auf dem zu benetzenden Substrat auf. Dies führt zu einer gleichmäßigen Benetzung der Oberfläche. So beträgt gemäß einer bevorzugten Ausführungsform der Erfindung der Kontaktwinkel der Zubereitung bei einer Temperatur von 23°C auf einem gereinigten Borosilikatglas nach einer Sekunde weniger als 28°, mehr bevorzugt weniger als 26°, mehr bevorzugt weniger als 24°, mehr bevorzugt weniger als 22°, mehr bevorzugt weniger als 20°, mehr bevorzugt weniger als 18°, mehr bevorzugt weniger als 15°, mehr bevorzugt weniger als 12°, mehr bevorzugt weniger als 10° und ganz besonders bevorzugt weniger als 5°. Hierbei nimmt mit zunehmenden Gehalt an Diluenten der Kontaktwinkel ab.

Alternativ oder zusätzlich weist gemäß einer anderen Ausführungsform ein Tropfen der Zubereitung mit einem Volumen von 10 µl bei 23°C, welcher auf einer gereinigten Borosilikatglasoberfläche aufgebracht wurde, eine Spreitung im Bereich von 7,3 und 20 mm, vorzugsweise im Bereich von 7,5 und 18 mm, insbesondere im Bereich von 7,9 und 17 mm, mehr bevorzugt im Bereich von 9 und 16 mm, mehr bevorzugt im Bereich von 9,5 und 15 mm, mehr bevorzugt im Bereich von 10 und 14 mm, mehr bevorzugt im Bereich von 11 und 13,5 mm und besonders bevorzugt im Bereich von 12 und 13 mm auf. Die Spreitung wurde hierbei 5s nach dem Auftragen des Tropfens durch Ausmessen der Tropfengröße mit Hilfe eines Lichtmikroskops bestimmt.

Der erfindungsgemäße, hohe Anteil an Diluenten in der Zubereitung wirkt sich auch vorteilhaft auf die Benetzungsgeschwindigkeit sowie die Vernetzungsgeschwindigkeit aus. So steigt mit der Konzentration des Diluenten in der Zubereitung die Vernetzungsgeschwindigkeit, so dass die Zubereitung auch schneller aufgetragen werden kann.

Die Zubereitung weist einen Diluentengehalt auf, welcher eine schnelle Vernetzung sowie einen homogenen Auftrag der Zubereitung auf das zu beschichtende Substrat sowie eine vorteilhafte Spreitung der Zubereitung auf dem Substrat gewährleistet. Dagegen können Zubereitungen, deren Diluentengehalt mehr als 95 Gew.-% beträgt, nicht mehr homogen aufgetragen werden. Entsprechende Zubereitungen weisen zudem keine messbare Spreitung mehr auf. Zudem weisen die nach dem Verdampfen des Diluenten erhaltenen Schichten nur eine sehr geringe Dicke auf, so dass diese Schichten nur eine unzureichende Gleitwirkung aufweisen. Zudem ist der Auftrag einer entsprechend verdünnten Zubereitung durch den zusätzlichen Anteil an Lösungsmittel, welcher sich nicht positiv auf die Schichteigenschaften auswirkt, unter wirtschaftlichen Aspekten nachteilig.

Weiterhin sind die erfindungsgemäßen Zubereitungen in Hinblick auf die enthaltene Silikonmenge sehr vorteilhaft. So kann die Konzentration der Silikonmischung über den Gehalt des Diluenten in der Zubereitung so eingestellt werden, dass die entsprechende Beschichtung die für die jeweiligen gewünschten Reibeigenschaften erforderliche, minimale Schichtdicke aufweist. Ein Auftrag an überschüssiger Silikonmischung wird somit ebenso verhindert wie zu dünne Schichten. Als besonders vorteilhaft haben sich hierbei Zubereitungen herausgestellt, bei denen der Anteil des Diluenten in der Zubereitung 45 Gewichtsprozent oder mehr und 95 Gewichtsprozent oder weniger, bevorzugt mehr als 45 Gewichtsprozent und weniger als 95 Gewichtsprozent, mehr bevorzugt 50 Gewichtsprozent oder mehr und weniger als 95 Gewichtsprozent, mehr bevorzugt 55 Gewichtsprozent oder mehr und weniger als 95 Gewichtsprozent, mehr bevorzugt 60 Gewichtsprozent oder mehr und weniger als 95 Gewichtsprozent, mehr bevorzugt 65 Gewichtsprozent oder mehr und weniger als 95 Gewichtsprozent, mehr bevorzugt 70 Gewichtsprozent oder mehr und weniger als 95 Gewichtsprozent, mehr bevorzugt 75 Gewichtsprozent oder mehr und weniger als 95 Gewichtsprozent, mehr bevorzugt 80 Gewichtsprozent oder mehr und 90 Gewichtsprozent oder weniger, meist bevorzugt 83 Gewichtsprozent oder mehr und 88 Gewichtsprozent oder weniger beträgt.

Gemäß einer Ausbildungsform der Erfindung weist die Zubereitung bei einer Temperatur von 23°C eine Viskosität im Bereich von 0,5 bis 200 mPas, bevorzugt 1 bis 50 mPas und besonders bevorzugt im Bereich von 1 bis 10 mPas auf.

Alternativ oder zusätzlich kann gemäß einer weiteren Ausführungsform die Zubereitung bei einer Temperatur von 23°C eine kinematische Viskosität im Bereich von 1 bis 50000 cSt, bevorzugt 10 bis 35000 cSt, mehr bevorzugt 100 bis 35000 cSt, mehr bevorzugt 2500 bis 22000 cSt, oder 5000 bis 35000 cSt, weiter bevorzugt 5000 bis 22000 cSt. Noch weiter bevorzugte Bereiche sind 5000 bis 18000 cSt, 5000 bis 15000 cSt, 5000 bis 10000 cSt. Ganz besonders bevorzugt sind dabei die Bereich von 5000 bis 5500 cSt, sowie 8000 bis 10000 cSt.

Ein Diluent im Sinne der Erfindung kann eine siliziumorganische Verbindung, bevorzugt ein Si-haltiges Lösungsmittel, mehr bevorzugt ein Si-haltiges, organisches Lösungsmittel sein, in welchem das reaktive Silikonsystem als auch das nichtreaktive Silikonöl löslich sind. Um eine gute Löslichkeit der erfindungsgemäßen Silikonkomponenten der Zubereitung zu gewährleisten, werden als Diluenten vorzugsweise unpolare Lösungsmittel verwendet. Als besonders vorteilhaft hat sich hierbei die Verwendung von siliziumorganischen Verbindungen mit höchstens 6 Siliziumatomen als Diluent herausgestellt.

Besonders geeignete Diluenten sind:
- Zyklische Silikone, insbesondere: Octamethylcyclotetrasiloxan, Decamethylcyclopentasiloxan, Dodecamethylcyclohexasiloxan, Tetramethylcyclotetrasiloxan, Pentamethylcyclopentasiloxan,
- Hexamethyldisiloxan (HMDSO),
- Octamethyltrisiloxan,
- Decamethyltetrasiloxan.

Als Diluent kann auch eine Mischung, insbesondere mit einem oder mehreren der vorstehend genannten Stoffe eingesetzt werden.

Das reaktive Silikon-System zur Ausbildung eines Silikonnetzwerkes weist Polysiloxane mit vernetzbaren Gruppen auf. Das reaktive Silikon-System ist bevorzugt ein Mehrkomponentensystem, besonders bevorzugt ein Zweikomponentensystem. Es sind sowohl hochtemperaturvernetzende (HTV-Systeme), als auch niedertemperatur- oder raumtemperaturvernetzende Silikon-Mehrkomponentensysteme (RTV-Systeme) getestet worden und geeignet. Das reaktive Silikonsystem enthält hierbei funktionelle Gruppen, welche eine Vernetzungsreaktion eingehen können.

Hierbei weist das reaktive Silikon-System vorzugsweise eine erste Komponente und eine zweite Komponente auf. Die erste Komponente weist dabei zumindest eine erste funktionelle Gruppe und die zweite Komponente mehrere zweite funktionelle Gruppen auf. Erste und zweite funktionelle Gruppen reagieren unter Bildung einer gemeinsamen Bindung miteinander. Die erste und die zweite funktionelle Gruppe können hierbei eine unterschiedliche oder aber auch dieselbe chemische Form aufweisen. Zum Beispiel kann es sich sowohl bei der ersten als auch bei den zweiten funktionellen Gruppen um Vinylgruppen handeln.

Vorzugsweise ist der Anteil der ersten Komponente am reaktiven Silikon-System höher als der Anteil der zweiten Komponente. Vorzugsweise beträgt das Massenverhältnis von erster zu zweiter Komponente 10:1 bis 30:1. Die erste Komponente bildet somit die Basis des bei der Vernetzung entstehenden Silikonnetzwerkes, während die zweite Komponente als Vernetzer fungiert.

Gemäß einer Weiterbildung dieser Ausführungsform umfasst die erste Komponente ein vinylfunktionalisiertes Polysiloxan und die zweite Komponente ein Polysiloxan mit Si-H-Gruppen. Eine bevorzugte Ausführungsform enthält als erste Komponente ein vinylfunktionalisiertes Polydimethylsiloxan und als zweite Komponente ein Copolymer mit Dimethylsiloxan- und Methylhydrosiloxan-Monomereinheiten. Als besonders vorteilhaft hat sich hierbei die Verwendung eines Copolymers mit folgender Struktur herausgestellt:

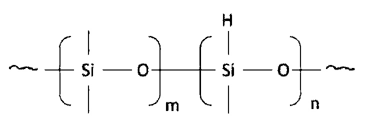

Durch das Verhältnis m/n und die Anordnung der beiden Monomereinheiten im Copolymer kann somit die Anzahl der Vernetzungsstellen in der Gleitschicht und somit der Vernetzungsgrad eingestellt werden.

Die Viskosität der Zubereitung wird weiterhin auch durch die Viskosität der enthaltenen Silikonöle beeinflusst. Gemäß einer Ausführungsform der Erfindung weist das nicht reaktive Silikonöl daher eine Viskosität im Bereich von 2.500 cSt bis 50.000 cSt, bevorzugt 5000 cSt bis 35.000 cSt und besonders bevorzugt 18.000 cSt bis 22.000 cSt auf.

Die Zubereitung enthält auch einen Katalysator für die Vernetzungsreaktion der Komponenten des Silikon-Mehrkomponentensystems. Besonders bevorzugt wird ein löslicher, Platin-haltiger Katalysator, beispielsweise Chlorplatinsäure verwendet.

Gemäß einer Ausführungsform der Erfindung beträgt der Anteil an Katalysator 0,001 - 5 Gew.-%, bevorzugt 0,01 -1,5 Gew.-% der Reaktionslösung. Als besonders vorteilhaft hat sich ein Gewichtsverhältnis von Katalysator zum reaktiven Silikonsystem im Bereich von 0,01 bis 0,2, bevorzugt im Bereich von 0,01 bis 0,1 herausgestellt. Hierbei kann über den Katalysatoranteil der Polymerisations- bzw. Vernetzungsgrad der Gleitschicht eingestellt werden. Bei zu geringen Mengen an Katalysator erfolgt die Polymerisations- bzw. Vernetzungsreaktion nur sehr langsam. Bei sehr hohen Katalysatormengen dagegen erfolgt die Vernetzung so schnell, dass unter Umständen die bei der Vernetzungsreaktion freigesetzte Reaktionswärme nicht mehr abgeführt werden kann und es so zu einer Verdampfung des Diluenten kommt. Somit nimmt die Menge an Diluent ab, so dass die Viskosität der Reaktionslösung steigt. Dies kann wiederum durch die Immobilisation der Polymerketten zu geringen Vernetzungs- bzw. Polymerisationsgraden führen. Die entsprechende Gleitschicht weist in diesem Fall nur eine geringe Stabilität auf.

Gemäß einer besonders bevorzugten Ausführungsform enthält die Zubereitung zumindest einen Inhibitor zur Unterbindung einer spontanen Reaktion des reaktiven Silikonsystems. Dies erleichtert die Handhabung der Zubereitung bis zum Auftrag der Gleitschicht. Zudem hat sich der Inhibitor nicht als nachteilig für die Gleiteigenschaften und die Festigkeit der Schicht erwiesen. Insbesondere haben sich organische Verbindungen mit Dreifachbindung als geeignete Inhibitoren herausgestellt. Der Inhibitor kann hierbei eine reversible Komplexbildung mit dem Katalysator eingehen, so dass eine spontane Vernetzungsreaktion des reaktiven Silikonsystems unterbunden wird.

Die Erfindung betrifft auch ein Verfahren zur Herstellung des Packmittels. Nach diesem Verfahren wird die Zubereitung auf der Innenseite des Hohlkörpers als Schicht aufgetragen und dann die Reaktion der Komponenten des Silikon-Mehrkomponentensystems in Gang gebracht, so dass sich ein Silikon-Netzwerk ausbildet und eine verfestigte Gleitschicht erhalten wird.

Das Auftragen der Zubereitung kann hierbei mittels einfacher Applikationsprozesse wie Sprühen oder Aufwischen auf die Behälterinnenwand erfolgen.

Die Aushärtung, d.h. die Vernetzung erfolgt vorzugsweise durch Erwärmung der aufgebrachten Schicht auf Temperaturen im Bereich von 150 bis 280°C. Die Temperaturbehandlung kann insbesondere über Infrarotstrahlung oder Konvektionsströme erfolgen. Durch die Temperaturbehandlung erfolgt die Vernetzungsreaktion im reaktiven Silikonöl-System. Gleichzeitig erfolgt eine zumindest teilweise Verdampfung des Teien. Alternativ kann die Aushärtung durch ein Plasma bei deutlich niedrigeren Temperaturen erfolgen.

Haltbare Gleitschichten bzw. Gleitfilme werden besonders erhalten, wenn die Schichtdicke der Gleitschicht weniger als 3 µm beträgt. Besonders bevorzugt werden Schichtdicken von kleiner als 1 µm. Damit ein hinreichender Vorrat an Silikonöl in der Schicht gespeichert werden kann und die Schicht durchgehend die vorgesehene Gleitfläche bedeckt, ist es weiterhin vorteilhaft, wenn die Schichtdicke mindestens 0,4 µm, bevorzugt mindestens 0,5 µm beträgt. Die entsprechenden Gleitschichten weisen hierbei eine hohe mechanische Stabilität auf. Zudem zeigen die Gleitschichten eine hohe Resistenz gegenüber Wasser sowie Chemikalien wie Ethanol oder Reinigungsmitteln. Unter einer hohen Resistenz im Sinne der Erfindung wird insbesondere verstanden, dass die Gleitreibung nach einer Behandlung der Gleitschicht mit den oben beschriebenen Substanzen nicht oder zumindest nicht dauerhaft ansteigt.

In überraschender Weise hat sich gezeigt, dass der mit dem erfindungsgemäßen Verfahren hergestellte Hohlkörper seine Haft-Gleitreibungs-Eigenschaften bei Abreinigung der Gleitschicht zumindest teilweise wieder regenerieren kann. So kann nach einer Abreinigung der Gleitschicht die ursprüngliche Haft-Gleitreibung durch Lagerung, beispielsweise unter Wärmeeinwirkung, z.B. direkt vor dem Setzen des Stopfens bzw. des Befüllens mindestens teilweise wiederhergestellt werden. Das Packmittel gemäß der Erfindung weist also eine Regenerationseigenschaft auf.

Gleitschichten, welche mit dem erfindungsgemäßen Verfahren, beziehungsweise mit der hier beschriebenen Zubereitung hergestellt werden, weisen also zusätzlich zu sehr geringen Haft- und Gleitreibungswerten die besondere Eigenschaft einer Regenerationsfähigkeit der Schicht in Bezug auf die Gleitwirkung auf, wenn die Oberfläche der Gleitschicht abgereinigt, beziehungsweise allgemein das zur Gleitwirkung wesentlich beitragende nicht reaktive Silikon oberflächlich entfernt wird.

Die Regenerationseigenschaft kann nachgewiesen werden, indem die Schichtoberfläche mit Ethylacetat gereinigt wird. Die Bestimmung der HGR-Werte wird vor und nach der Reinigung sowie gegebenenfalls nach einer regenerativen Behandlung (z.B. Lagern, gegebenenfalls bei erhöhter Temperatur) vorgenommen.

Die Erfindung betrifft des Weiteren auch ein Packmittel für Pharmazeutika oder kosmetische Produkte, welches einen zylindrischen Hohlkörper umfasst, der innenseitig mit einer Gleitschicht beschichtet ist, wobei die Gleitschicht ein Silikon-Netzwerk aufweist, in welchem ein Silikonöl aufgenommen ist, wobei der Hohlkörper derart ausgestaltet ist, dass ein Stopfen in den Hohlkörper eingeführt werden kann und der Haftreibungswert der Reibung eines in den Hohlkörper eingesetzten Stopfens an der Gleitschicht maximal 20% größer, bevorzugt maximal 10% größer, besonders bevorzugt maximal 5% größer als der mittlere Gleitreibungswert ist. Somit sind bei diesen Gleitschichten Haft- und Gleitreibungswerte nahezu identisch, während Systeme gemäß dem Stand der Technik eine verglichen mit der mittleren Gleitreibung deutlich größere Haftreibungswerte im Verhältnis zum Gleitreibungswert aufweisen. Dies kann beispielsweise dazu führen, dass Stopfen, welche auf der Gleitschicht bewegt werden, keine gleichmäßige Bewegung erfahren, sondern nach Überwindung der Haftreibung unkontrolliert und schnell eingeschoben werden. Das erfindungsgemäße Packmittel ermöglicht hingegen eine sehr gleichmäßige und kontrollierte Bewegung des Stopfens durch den Hohlkörper.

Die Haft- und Gleitreibungswerte werden ermittelt, indem ein geeigneter Stopfen direkt nach dem Einsetzen in den Hohlkörper mit einer konstanten Geschwindigkeit von 100 mm/min durch den Hohlkörper bewegt wird und die dafür erforderliche Kraft als Funktion der Einschubtiefe gemessen wird. Typische Haft-Gleitreibungsdiagramme weisen zu Beginn der Bewegung des Stopfens einen linearen Kraftanstieg auf. Sobald die Haftreibungskraft überwunden ist, setzt sich der Stopfen in Bewegung und es kommt zu einem Gleiten des Stopfens durch den Zylinder, welche bei einer guten Gleitschicht eine relativ konstante Schubkraft erfordert. Direkt vor Einsetzen der Gleitbewegung des Stopfens tritt in der Regel ein Maximum in dem Haft-Gleitreibungsdiagramm auf, welches den Haftreibungswert darstellt. Die relativ konstante Kraft während der Gleitbewegung stellt den Gleitreibungswert dar. Die Haft-Gleitreibungsdiagramme sollten typischerweise mit einem Stopfen gemessen werden, der auch als Stopfen für das pharmazeutische Primärpackmittel eingesetzt wird und mit welchem eine entsprechende Dichtigkeit erreicht wird, ohne jedoch zu großen Kraftaufwand zu benötigen. Bevorzugt kommt ein Standardstopfen zum Einsatz. Ein Standardstopfen im Sinne dieser Anmeldung besteht aus einem Elastomer, welches durch eine Vernetzung/Vulkanisation polymerisiert ist. Ein Standardstopfen kann mit einer sehr dünnen Silikonschicht überzogen sein, da Stopfen üblicherweise in Formen vulkanisiert werden, welche zum Entformen mit einer Antihaftbeschichtung beispielsweise aus Silikon beschichtet sind. Ein Standardstopfen weist ferner einen im Vergleich zu dem Innendurchmesser des Gleitkolbens etwas größeren Außendurchmesser auf, so dass er im eingesetzten Zustand unter Kompression steht. So kann z.B. der Stopfen bei einem typischen Innendurchmesser des Gleitkolbens einer Spritze des Formates «1ml long» von 6,5 mm einen Außendurchmesser von 6,9 +/- 0,1 mm aufweisen.

Gemäß einer vorteilhaften Ausgestaltung der Erfindung ist der Haftreibungswert und/oder der Gleitreibungswert bei einer Bewegung eines Standardstopfens mit einer Geschwindigkeit von 100 mm/min über die Gleitschicht kleiner als 10 N, bevorzugt kleiner als 8 N oder sogar kleiner als 6 N.

Die Erfinder haben erkannt, dass das erfindungsgemäße Packmittel überraschender Weise einen Regenerationseffekt aufweist. Gemäß einer Ausführungsform der Erfindung weist die Gleitschicht daher einen Regenerationseffekt auf, so dass sich nach einer Entfernung des Silikonöls von der Oberfläche der Gleitschicht die Gleitwirkung der Gleitschicht regeneriert. Es ist ersichtlich, dass ein derartiges Packmittel sehr vorteilhaft ist, wenn beispielsweise vor dem Befüllen des Packmittels mit dem pharmazeutischen oder kosmetischen Erzeugnis eine Reinigung vorangeht. Hierbei wird im Sinne der Erfindung eine Gleitschicht insbesondere dann als Gleitschicht mit Regenerationseffekt bezeichnet, wenn nach einer Anhebung der Gleitreibung von einem Ausgangsniveau auf ein höheres Niveau, welches in einem Zeitraum von weniger als 5 Minuten nach einem Abreinigen des Silikonöls bestimmt wird, die Gleitreibung nach einem Regenerationszeitraum von mehr als 10 Minuten nach dem Abreinigen des Silikonöls wieder signifikant abfällt. Unter einem Regenerationseffekt wird hierbei auch ein Abfall der Gleitreibung nach dem Abreinigen verstanden, wenn die Gleitreibung nach dem Abreinigen des Silikonöls und anschließender Regenerationszeit nicht wieder das Ausgangsniveau, d.h. den Reibungswert vor dem Abreinigen des Silikonöls erreicht.

Die Gleitschicht ist besonders geeignet für Behälter aus Kunststoff, insbesondere für Behälter aus Polyolefin, wie Cycloolefin-Copolymer (COC) oder Cycloolefin-Polymer (COP). Ein weiteres geeignetes Material ist Glas, insbesondere ein Borosilikatglas, vorzugsweise ein Glas der hydrolytischen Klasse 1b gemäß ISO 719. Eine weitere Klasse geeigneter Gläser sind Alumosilikatgläser. Als Alumosilikatgläser werden silikatische Gläser mit einem Gehalt von Al₂O₃ von mehr als 6 Gewichtsprozent bezeichnet.

Überraschenderweise kann eine haltbare Gleitschicht auch auf Polyolefinsubstrate wie beispielsweise COC oder COP aufgebracht werden, obwohl Polyolefine kein Silizium enthalten und somit auch keine kovalente Anbindung des Silikon-Netzwerks an das Substrat durch Si-Si oder Si-O-Bindungen erfolgen kann. Ohne an diese Hypothese gebunden sein zu wollen, vermuten die Erfinder, dass der siliziumhaltige Diluent eine Vermittlerrolle zwischen dem unpolaren, siliziumfreien Substrat und dem Silikon-Netzwerk erfüllt. Gemäß einer Ausführungsform der Erfindung ist der Behälter daher aus einem Silizium-freien Material gefertigt. Überraschender Weise ist weder bei Silizium-freien Materialien, noch bei Silizium enthaltenden Behältermaterialien, wie Borosilikatglas oder Aluminosilikatglas ein Primer erforderlich, um die Gleitschicht aufzutragen und haltbar zu verfestigen. Gemäß noch einer Ausführungsform der Erfindung wird daher die Zubereitung ohne Vorbehandlung mit einem Primer direkt auf die Behälterinnenseite aufgetragen und dann verfestigt. Die Gleitschicht kann somit unmittelbar auf der inneren Oberfläche des Behälters aufgebracht werden.

Gemäß einer Weiterbildung der Erfindung kann die Rauigkeit einer Kunststoffoberfläche durch die erfindungsgemäße Schicht vermindert werden. Die Rauheit der Gleitschicht ist hierbei kleiner 40 nm, bevorzugt kleiner 20 nm.

Als nicht reaktives Silikonöl eignet sich besonders Polydimethylsiloxan. Generell ist es beim nicht reaktiven Silikonöl von Vorteil, wenn die Kettenlänge des Silikonöls nicht zu groß ist, da sich dies vorteilhaft auf die Regenerationsfähigkeit der Gleitschicht auswirkt. Das Polydimethylsiloxan weist eine mittlere Kettenlänge, beziehungsweise dementsprechend eine mittlere Molmasse auf, die so gering ist, dass es bei Raumtemperatur von 23°C noch als Öl vorliegt, also fluid ist.

Die Gleitfläche kann besonders in einer Spritze oder Karpule angeordnet sein, um das Gleiten eines Stopfens, beziehungsweise Kolbens für das Aufziehen und/oder Abgeben des pharmazeutischen oder kosmetischen Produkts zu erleichtern. Gemäß einer Ausführungsform ist daher vorgesehen, dass das Packmittel eine Spritze oder Karpule ist. Dabei bedeckt die Gleitschicht vorzugsweise zumindest den Bereich der Innenseite des Hohlkörpers, über den der Stopfen gleiten kann.

Dieses Merkmal folgt offensichtlich aus der speziellen Zubereitung mit dem hohen Anteil an Diluenten unabhängig von einer Regenerationsfähigkeit der Gleitschicht nach Abreinigen des Silikonöls.

In einer bevorzugten Ausführungsform weisen die Gleitschichten ein Verhältnis µ_{G}/ µ_{H} von Gleitreibungskoeffizienten µ_{G} zum Haftreibungskoeffizienten µ_{H} von mehr als 0,8 auf. Bevorzugt beträgt das Verhältnis µ_{G}/ µ_{H} zumindest 0,9 oder sogar 0,95. Somit sind bei diesen Gleitschichten Haft- und Gleitreibungen nahezu identisch, während aus dem Stand der Technik eine, verglichen mit der Haftreibung, größere Haftreibung aufweisen. Dies kann beispielsweise dazu führen, dass Stopfen, welche auf der Gleitschicht bewegt werden, keine gleichmäßige Bewegung erfahren, sondern gestoppt werden.

Die Erfindung wird nachfolgend weiter anhand von Ausführungsbeispielen und den beigeschlossenen Figuren erläutert.

### Kurzbeschreibung der Figuren:

Fig. 1 zeigt ein Packmittel für pharmazeutische oder kosmetische Produkte in Form einer Spritze.
Fig. 2 zeigt ein Diagramm mit Reibungswerten der Stopfenverschiebung für zehn Spritzen.
Fig. 3 zeigt ein Diagramm mit Reibungswerten der Stopfenverschiebung nach einer Behandlung der Spritze für 60 s in Wasser mittels Ultraschall.
Fig. 4 zeigt ein Diagramm mit Reibungswerten der Stopfenverschiebung nach einer Behandlung der Spritze für 60 s in einer 0,1 M wässrigen NaOH-Lösung, Lagerzeit vor Messung war 30 min.
Fig. 5 zeigt ein Diagramm mit Reibungswerten der Stopfenverschiebung nach einer Behandlung der Spritze für 60 s in Aceton mittels Ultraschall.
Fig. 6 zeigt ein Diagramm mit Reibungswerten der Stopfenverschiebung nach Entfernung des nicht reaktiven Silikonöls aus der Gleitschicht (über 60 s Ultraschallbehandlung mit Ethylacetat).
Fig. 7 zeigt ein Diagramm mit Reibungswerten der Stopfenverschiebung nach Entfernung des nicht reaktiven Silikonöls aus der Gleitschicht und einer sich daran anschließenden Lagerung für 24 h bei Raumtemperatur.
Fig. 8 zeigt ein Diagramm mit Reibungswerten der Stopfenverschiebung nach Entfernung des nicht reaktiven Silikonöls aus der Gleitschicht und einer sich daran anschließenden Lagerung für 24 h bei 40°C.

### Beschreibung der Figuren und Ausführungsbeispiele

### Ausführungsbeispiel 1

In einem ersten Ausführungsbeispiel wurden in einem Reaktionsgefäß 10 g eines vinylfunktionalisierten Polydimethylsiloxans vorgelegt und 65 g Decamethylcyclopentasiloxan versetzt. Unter ständigem Rühren bei 800 rpm wurden diesem Reaktionsgemisch 0,5 g Methylhydrosiloxan-Dimethylsiloxane Copolymer, 6,25 g flüssiges Polydimethylsiloxan, 0,01 g 10% ige Hexachloridoplatinsäure in Isopropanol als Katalysator und 0,05 g des 2,4,7,9-Tetramethyl-5-decyne-4,7-diol als Inhibitor zugesetzt. Die Reaktionslösung ist nach einer Rührzeit von 60 s einsatzfähig. Hierbei bilden das vinylfunktionalisierte Polymethylsiloxan und das Methylhydrosiloxan-Dimethylsiloxane Copolymer das reaktive Silikonsystem, das Polydimethylsiloxan das nicht reaktive Silikonöl und Decamethylcyclopentasiloxan den Diluenten.

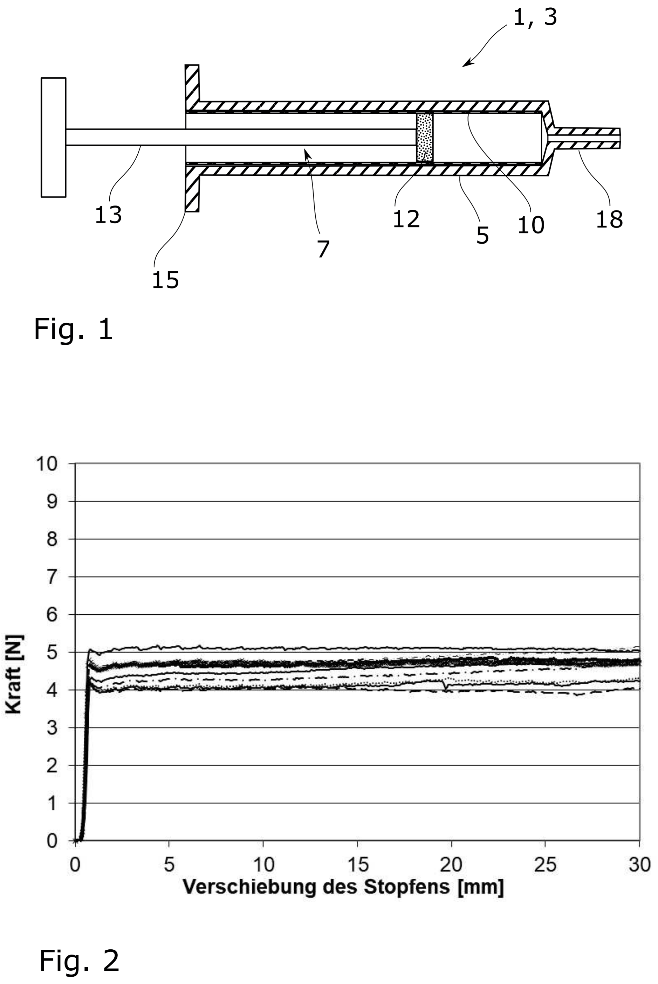

Die so hergestellte Zubereitung wurde mittels eines Wischprozesses auf den Kunststoffhohlkörper auf der Innenseite aufgebracht und mittels einer Erhitzung auf 175 °C für 20 s ausgehärtet. Hierbei wurde als Substrat zur Abscheidung der Gleitschicht eine 1 ml COC-Spritze der Standardgröße "1ml long" mit einem Innendurchmesser von 6,5 mm verwendet. Anschließend wurden Haft- und Gleitreibungswerte der ausgehärteten Gleitschicht bestimmt. Hierbei wurde der Stopfen V9361 FM457/0 FLNC2 057 der Firma Datwyler Pharma Packaging.mit einem Außendurchmesser von 6,9 +/- 0,1 mm mit einer Geschwindigkeit von 100 mm/ min in die Spritze gedrückt. Die dabei notwendigen Kräfte wurden aufgezeichnet. Sowohl die Haft- als auch die Gleitreibungswerte waren dabei kleiner 10 N. Das Ergebnis der Messung ist in Fig. 2 gezeigt.

### Ausführungsbeispiel 2

In einem zweiten Ausführungsbeispiel wurden in einem Reaktionsgefäß 80 g eines vinylfunktionalisierten Polydimethylsiloxans vorgelegt und mit 640 g Hexamethyldisiloxan versetzt. Unter ständigem Rühren bei 1000 rpm wurden diesem Reaktionsgemisch 2 g Methylhydrosiloxan-Dimethylsiloxane Copolymer, 48 g flüssiges Polydimethylsiloxan, 1,1 g 1,1,3,3-Tetramethyl-1,3-divinyldisiloxan-komplexiertes Platin und 0,1 g Butinol als Inhibitor zugesetzt. Die Zubereitung ist nach einer Rührzeit von 60 s einsatzfähig.

Die Zubereitung wurde mittels eines Wischprozesses auf den Kunststoffhohlkörper auf der Innenseite aufgebracht und mittels einer Erhitzung auf 250 °C für 3,5 s ausgehärtet. Hierbei wurde als Substrat zur Abscheidung der Gleitschicht eine 1 ml COC-Spritze der Standardgröße "1ml long" mit einem Innendurchmesser von 6,5 mm verwendet. Anschließend wurden Haft- und Gleitreibungswerte der ausgehärteten Gleitschicht bestimmt. Hierbei wurde der Stopfen V9361 FM457/0 FLNC2 057 der Firma Datwyler Pharma Packaging.mit einem Außendurchmesser von 6,9 +/- 0,1 mm mit einer Geschwindigkeit von 100 mm/ min in die Spritze gedrückt. Die dabei notwendigen Kräfte wurden aufgezeichnet. Sowohl die Haft- als auch die Gleitreibungswerte waren dabei kleiner 10 N.
Tabelle 1 zeigt die Rauheitswerte einer unbeschichteten Spritze aus COC sowie die Rauheit der Oberfläche der entsprechenden Spritze nach Aufbringen einer erfindungsgemäßen Gleitschicht.

**Tabelle 1: Vergleich der Rauheitswerte**

| Probe | Bereich | Rms nm | Ra nm |
|---|---|---|---|
| beschichtete COC-Spritze, 20 ml nicht steril | nahe Konus | 10 | 7 |
| | Mitte | 12 | 9 |
| | nahe Flansch | 29 | 24 |
| nicht beschichtete COC-Spritze, 20 ml | nahe Konus | 19 | 14 |
| | Mitte | 40 | 27 |
| | nahe Flansch | 79 | 62 |

Die Rauigkeitswerte wurden gemäß der DIN EN ISO/IEC 17025 mit einem Weißlichtinterferrometer bestimmt.

### Ausführungsbeispiel 3

In einem dritten Ausführungsbeispiel wurden in einem Reaktionsgefäß 80 g eines vinylfunktionalisierten Polydimethylsiloxans vorgelegt und mit 640 g Decamethylcyclopentasiloxan versetzt. Unter ständigem Rühren bei 1000 rpm wurden diesem Reaktionsgemisch 2 g Methylhydrosiloxan-Dimethylsiloxane Copolymer, 48 g flüssiges Polydimethylsiloxan, 1,1 g 10% ige Hexachloridoplatinsäure in Isopropanol als Katalysator und 0,1 g Butinol als Inhibitor zugesetzt. Die Zubereitung ist nach einer Rührzeit von 60 s einsatzfähig.

Die Zubereitung wurde mittels eines Wischprozesses auf den Kunststoffhohlkörper auf der Innenseite aufgebracht und mittels einer Erhitzung auf 250 °C für 3,5 s ausgehärtet. Hierbei wurde als Substrat zur Abscheidung der Gleitschicht eine 1 ml COC-Spritze der Standardgröße "1ml long" mit einem Innendurchmesser von 6,5 mm verwendet. Anschließend wurden Haft- und Gleitreibungswerte der ausgehärteten Gleitschicht bestimmt. Hierbei wurde der Stopfen V9361 FM457/0 FLNC2 057 der Firma Datwyler Pharma Packaging.mit einem Außendurchmesser von 6,9 +/- 0,1 mm mit einer Geschwindigkeit von 100 mm/ min in die Spritze gedrückt. Die dabei notwendigen Kräfte wurden aufgezeichnet. Sowohl die Haft- als auch die Gleitreibungswerte waren dabei kleiner 10 N.

Die erfindungsgemäße Gleitschicht weist eine geringe Rauheit auf. Insbesondere beträgt die Rauheit Rms der Gleitschicht höchstens 40 nm, bevorzugt höchstens 30 nm und besonders bevorzugt höchstens 20 nm. Durch die geringe Rauheit können besonders niedrige Haft- und Gleitreibungswerte erzielt werden. Darüber hinaus kann gemäß einer Weiterbildung der Erfindung die Rauheit der verwendeten Substrate durch das Aufbringen einer erfindungsgemäßen Gleitschicht verringert werden. Dies ist insbesondere bei Verwendung von Kunststoffsubstraten wie beispielsweise COC-Substraten, welche verglichen mit Glas eine größere Rauheit aufweisen, vorteilhaft. Gemäß einer Ausführungsform dieser Weiterbildung weist ein mit der erfindungsgemäßen Gleitschicht beschichtetes Substrat eine um zumindest 20%, bevorzugt zumindest 40% und besonders bevorzugt zumindest 40% verringerte Rauheit Rms gegenüber dem entsprechenden unbeschichteten Substrat auf.

Nachfolgend soll an Hand von Vergleichsbeispielen der Einfluss der einzelnen Komponenten auf die Zubereitungs- bzw. Gleitschichteigenschaften gezeigt werden.

### Vergleichsbeispiel 1: Einfluss des Gehaltes des Diluenten

In einem Reaktionsgefäß wurden 80 g eines vinylfunktionalisierten Polydimethylsiloxans vorgelegt und mit 80 g Hexamethyldisiloxan versetzt. Unter ständigem Rühren bei 400 rpm wurden diesem Reaktionsgemisch 2 g Methylhydrosiloxan-Dimethylsiloxane Copolymer, 48 g flüssiges Polydimethylsiloxan , 1,1 g 10% ige Hexachloridoplatinsäure in Isopropanol als Katalysator und 0,1 g Butinol als Inhibitor zugesetzt. Die Reaktionslösung ist nach einer Rührzeit von 60 s einsatzfähig.

Die Reaktionslösung wurde mittels eines Wischprozesses auf den Kunststoffhohlkörper auf der Innenseite aufgebracht und mittels einer Erhitzung auf 250 °C für 3,5 s versucht auszuhärten.

Die Reaktionslösung verblieb jedoch nicht vollständig im Kunststoffhohlkörper in aufrechter Lagerung mit der Öffnung nach unten / bzw. sammelte sich bei waagerechter Lagerung unten im Kunststoffhohlkörper. Auf eine Haft- oder Gleitreibwertmessung wurde aufgrund der hohen Inhomogenität der Schicht im Kunststoffhohlkörper verzichtet.

### Vergleichsbeispiel 2 (Einfluss des Diluenten)

In diesem Vergleichsbeispiel wurde als Diluent Toluol verwendet, welches sich unter den Diluenten auszeichnet durch seinen unpolaren Charakter, ohne Si-Atome zu enthalten. So wurden in einem Reaktionsgefäß 80 g eines vinylfunktionalisierten Polydimethylsiloxans vorgelegt und mit 640 g Toluol versetzt. Unter ständigem Rühren bei 400 rpm wurden diesem Reaktionsgemisch 2 g Methylhydrosiloxan-Dimethylsiloxane Copolymer, 48 g flüssiges Polydimethylsiloxan, 1,1 g 1,1,3,3-Tetramethyl-1,3-divinyldisiloxan-komplexiertes Platin als Katalysator und 0,1 g Butinol als Inhibitor zugesetzt. Die Reaktionslösung ist nach einer Rührzeit von 60 s einsatzfähig.

Die Zubereitung wurde mittels eines Wischprozesses auf den Kunststoffhohlkörper auf der Innenseite aufgebracht und mittels einer Erhitzung auf 250 °C für 3,5 s versucht auszuhärten.

Die Zubereitung zeigte starke Benetzungsfehler und bildete keinen homogenen Film aus. Eine starke Tropfenbildung war sichtbar. Die Reaktionslösung härtete nicht vollständig aus. Auf eine Haft- oder Gleitreibwertmessung wurde aufgrund der hohen Inhomogenität der Schicht im Kunststoffhohlkörper verzichtet. Es wird somit deutlich, dass der Diluent nicht nur bezüglich seiner Kompatibilität, d.h. der Löslichkeit der einzelnen Zubereitungskomponenten ausgewählt werden kann.

### Vergleichsbeispiel 3 (Einfluss des Diluenten)

Vergleichbare Ergebnisse wurden auch beim dritten Vergleichsbeispiel erhalten.

In einem Reaktionsgefäß wurden 80 g eines vinylfunktionalisierten Polydimethylsiloxans vorgelegt und 640 g Cyclohexan versetzt. Unter ständigem Rühren bei 400 rpm wurden diesem Reaktionsgemisch 2 g Methylhydrosiloxan-Dimethylsiloxane Copolymer, 48 g flüssiges Polydimethylsiloxan, 1,1 g 1,1,3,3-Tetramethyl-1,3-divinyldisiloxan-komplexiertes Platin als Katalysator zugesetzt.

Die Reaktionslösung wurde mittels eines Wischprozesses auf den Kunststoffhohlkörper auf der Innenseite aufgebracht und mittels einer Erhitzung auf 250 °C für 3,5 s versucht auszuhärten.

Die Reaktionslösung zeigte starke Benetzungsfehler und bildete keinen homogenen Film aus. Eine Tropfen- und Schlierenbildung konnte beobachtet werden. Die Reaktionslösung härtete nicht vollständig aus. Auf eine Haft- oder Gleitreibwertmessung wurde aufgrund der hohen Inhomogenität der Schicht im Kunststoffhohlkörper verzichtet.

### Vergleichsbeispiel 4 (Katalysatormenge)

In einem Reaktionsgefäß wurden 80 g eines vinylfunktionalisierten Polydimethylsiloxans vorgelegt und mit 640 g Toluol versetzt. Unter ständigem Rühren bei 400 rpm wurden diesem Reaktionsgemisch 2 g Methylhydrosiloxan-Dimethylsiloxane Copolymer, 48 g flüssiges Polydimethylsiloxan, 11 g 1,1,3,3-Tetramethyl-1,3-divinyldisiloxan-komplexiertes Platin als Katalysator zugesetzt. Der Anteil des Katalysators in diesem Vergleichsbeispiel beträgt 1,4 Gew.-%, Das Verhältnis zwischen dem Gewicht des Katalysators und dem Gewicht des reaktiven Silikonsystems beträgt 1: 7,45.

Aufgrund des stark erhöhten Anteils an Katalysator zeigte die Zubereitung eine stark exotherme Reaktion unter Ausbildung von Gasen. Hierbei kann vermutet werden, dass es sich um gasförmiges Toluol handelte. Die Zubereitung gelierte und die Aufbringung eines Films war nicht möglich.

Die Zusammensetzungen weiterer Ausführungs- und Vergleichsbeispiele sind in Tabelle 2 aufgeführt.

**Tabelle 2: Zusammensetzung der Beispiele A bis E**

| Beispiel | vinylfunktionalisiertes Polydimethylsiloxan [Gew.-%] | Methylhydrosiloxan-Dimethylsiloxan-Copolymer [Gew.-%] | 10%ige Hexachloridoplatinsäure in Isopropanol [Gew.-%] | Polydimethylsiloxan (flüssig) [Gew.-%] | HMDSO [Gew.-%] |
|---|---|---|---|---|---|
| A | 41,69 | 1,04 | 0,59 | 16,68 | 40,00 |
| B | 38,22 | 0,96 | 0,54 | 15,29 | 45,00 |
| C | 10,59 | 0,26 | 0,15 | 4,24 | 84,75 |
| D | 3,47 | 0,09 | 0,05 | 1,39 | 95,00 |
| E | 0,69 | 0,02 | 0,01 | 0,28 | 99,0 |

Bei den Beispielen B bis D handelt es sich um Ausführungsbeispiele, während Beispiel A ein Vergleichsbeispiel für eine Zubereitung mit einem geringerem Anteil an Diluent (40 Gew.-%) und Beispiel E ein Vergleichsbeispiel für eine Zubereitung mit einem höheren Anteil an Diluent (99 Gew.-%) als die erfindungsgemäßen Zubereitungen darstellt.

Tabelle 3 zeigt die Messergebnisse zur Bestimmung des Spreitungsverhaltens der einzelnen Beispiele A bis E. Hierzu wurde jeweils ein Tropfen der entsprechenden Zubereitung mit einem Volumen von 10 µl bei einer Temperatur von 23°C auf ein gereinigtes Borosilikatflachglas aufgebracht. Die Tropfengröße, d.h. die lateralen Abmaße des Tropfens auf der Glasoberfläche, wurde mit Hilfe eines Lichtmikroskops ausgemessen. Hierbei wurde die erste Messung (t₀) schnellstmöglich nach Ausbildung des Tropfens, d.h. innerhalb von 1 s nach dem Aufbringen des Tropfens auf die Glasoberfläche, durchgeführt.

**Tabelle 3: Spreitungsverhalten**

| Beispiel | Spreitung bei t₀ [mm] | Spreitung nach 5s [mm] | Spreitung nach 30 s [mm] | Spreitung nach 60 s [mm] | Spreitung nach 120 s [mm] |
|---|---|---|---|---|---|
| A | 7,0 | 7,4 | 8,1 | 8,3 | 8,4 |
| B | 7,5 | 7,9 | 8,8 | 9,0 | 9,0 |
| C | 12,2 | 12,4 | 13,1 | 13,0 | 13,2 |
| D | 7,3 | 7,5 | 7,6 | 7,5 | 7,6 |
| E | 7,8 | 7,2 | 3,4 | - | - |

Die in Tabelle 3 angegebenen Zeiten bezeichnen dabei die Zeitspanne zwischen t₀ und der Bestimmung der Ausbreitung bzw. Spreitung des Tropfens auf der Glasoberfläche. Für Beispiel E könnte nach 60s keine Spreitung mehr bestimmt werden, da der Großteil der Zubereitung zu diesem Zeitpunkt bereits verdunstet war.

Tabelle 4 zeigt die Ergebnisse der Tropfenkonturanalyse zur Bestimmung des Kontaktwinkels.

**Tabelle 4: Kontaktwinkel**

| Beispiel | Kontaktwinkel bei t₀ | Kontaktwinkel nach 1 s | Kontaktwinkel nach 2 s | Kontaktwinkel nach 3 s |
|---|---|---|---|---|
| A | 48,5° | 28° | 21° | 17° |
| B | 48° | 22,75° | 18,25° | 16° |
| C | <5° | <5° | <5° | <5° |
| D | <5° | <5° | <5° | <5° |
| E | <5° | <5° | <5° | <5° |

Zur Bestimmung des Kontaktwinkels wurde die Tropfenkonturanalyse (Drop Shape Analysis, DSA) verwendet. Die Tropfenkonturanalyse ist hierbei eine Bildanalysemethode zur Bestimmung des Kontaktwinkels aus dem Schattenbild eines auf einer Glasoberfläche liegenden Tropfens. Hierbei wurde jeweils ein Tropfen der Zubereitung bei einer Temperatur von 23°C auf ein gereinigtes Borosilikatflachglas aufgebracht. Mit Hilfe einer Kamera wurde ein Bild des Tropfens aufgenommen und diese in die Tropfenkonturanalysesoftware DSA übertragen. Die Konturerkennung wurde anhand einer Graustufenanalyse des Bildes vorgenommen und anschließend eine die Kontur des Tropfens beschreibendes, geometrisches Modell an die Tropfenkontur gefittet. Aus diesem wurde der Kontaktwinkel als der Winkel zwischen der Tropfenkontur und der Probenoberfläche ermittelt.

### Figurenbeschreibung

In Fig. 1 ist ein Packmittel 1 in einer für die Erfindung besonders bevorzugten Ausführung als Spritze 3 zur Verabreichung von Pharmazeutika oder Kosmetika gezeigt. Die Spritze 3, beispielsweise aus Glas oder vorzugsweise aus Kunststoff ist ein Hohlkörper 5 mit einem zylindrischen Abschnitt 7 und einem Luer-Konus 18, auf den z.B. eine Injektionsnadel aufgesetzt werden kann. Im zylindrischen Abschnitt ist ein Stopfen 12 eingesetzt, der durch Druck auf eine Schubstange 13 in axialer Richtung verschiebbar ist. Zur Handhabung weist der zylindrische Abschnitt am Ende der Einführöffnung für den Stopfen 12 einen Flansch 15 auf.

Das Packmittel 1 ist innenseitig, hier speziell auf der Innenseite des zylindrischen Abschnitts 7 mit einer Gleitschicht 10 versehen. Damit bedeckt die Gleitschicht den Bereich der Innenseite des Hohlkörpers 5, über den der Stopfen 12 bei der Entleerung der Spritze oder beim Aufziehen gleiten kann.

Die Gleitschicht 10 ist als Silikon-Netzwerk ausgebildet, in welchem ein Silikonöl aufgenommen ist.

Eine Gleitschicht 10, wie sie mit der hier beschriebenen Zubereitung und dem Verfahren hergestellt werden kann, zeichnet sich im Allgemeinen auch dadurch aus, dass die Reibungswerte bei der Bewegung des Stopfens nicht nur niedrig sind, sondern auch sehr gleichmäßig. Dies betrifft sowohl die Variation der Reibungswerte entlang des Verschiebungswegs des Stopfens, als auch die Unterschiede zwischen verschiedenen Packmitteln.

Dies belegt das Diagramm der Fig. 2. Im Diagramm sind die Reibungswerte bei der Bewegung des Stopfens für zehn verschiedene Spritzen dargestellt. Die Reibungskraft wurde für den gesamten möglichen Verschiebungsweg aufgezeichnet. Für die Messung der Haft-Gleitreibungs-Kurven wurden ein Stopfen in die Spritze eingesetzt und dann die Haft-Gleitreibungs-Kurve aufgenommen. Wie dargestellt liegen die Schwankungen zwischen den verschiedenen getesteten Exemplaren bei maximal etwa einem Newton, wobei hier auch Toleranzen im Material und den Abmessungen des Stopfens wesentlich beitragen. Zudem geht aus Fig. 2 hervor, dass alle vermessenen Spritzen ein Verhältnis µ_{G}/ µ_{H} der Reibungskoeffizienten von mehr als 0,95 aufweisen. Die Ergebnisse sind in Tabelle 5 zusammengefasst.

**Tabelle 5: Haft- und Gleitreibungswerte der in Fig. 2 dargestellten Messungen**

| Probe | FGleitreibung [N] | F_{Haftreibung}max [N] | µ_{G}/ µ_{H} |
|---|---|---|---|
| 1 | 4,49 | 4,33 | 1,03 |
| 2 | 4,13 | 4,20 | 0,98 |
| 3 | 4,26 | 4,36 | 0,98 |
| 4 | 4,73 | 4,53 | 0,96 |
| 5 | 4,62 | 4,73 | 0,98 |
| 6 | 4,22 | 4,41 | 0,96 |
| 7 | 4,87 | 4,61 | 1,06 |
| 8 | 4,34 | 4,24 | 1,02 |
| 9 | 4,04 | 4,28 | 0,94 |
| 10 | 5,19 | 4,85 | 1,07 |

Hierbei kann das Verhältnis µ_{G}/µ_{H} direkt aus den in Tabelle 5 aufgeführten Reibungswerten abgelesen werden. So ist die gemessene Kraft unmittelbar zu Beginn des Verschiebungsvorganges des Stopfens der Haftreibung zuzuordnen, die durchschnittliche Gleitreibung kann der gemessenen Kraft während des Verschiebungsvorgangs zugeordnet werden. Die in Tabelle 5 aufgeführte Kraft F_{Gleitreibung} stellt hierbei den Wert für die Gleitreibung gemittelt über alle Messwerte die bei Verschiebung des Stopfens im Bereich von 3 bis 30 mm erhalten wurden. Durch Division der beiden Kraftwerte kann somit das Verhältnis µ_{G}/ µ_{H} ermittelt werden.

Noch deutlich geringer sind die Schwankungen jeweils entlang des Verschiebeweges, wenn man nur eine einzige Spritze betrachtet. Die Gleitreibung schwankt in allen Fällen entlang des Wegs um weniger als 0,5 Newton. Dies ist vorteilhaft, um eine gleichmäßige Bewegung und einen gleichmäßigen Kraftaufwand bei der Abgabe des Inhalts des Packmittels zu gewährleisten. Die Gleitreibung liegt bei den gezeigten Beispielen zwischen 4 Newton und 5 Newton. Zwar hängt die absolute Größe der Reibungskraft auch von den Abmessungen ab, aber eine davon im Wesentlichen unabhängige Größe ist die relative Schwankung. Ohne Beschränkung auf das speziell dargestellte Ausführungsbeispiel ist dazu gemäß einer Ausführungsform vorgesehen, dass die Reibung entlang des verschiebbaren Wegs des Stopfens um höchstens ein Zehntel des Mittelwerts der Reibung schwankt.

Auffallend ist auch, dass die Kurven zu Beginn der Verschiebung praktisch keine Überhöhung aufgrund einer erhöhten Haftreibung zeigen.

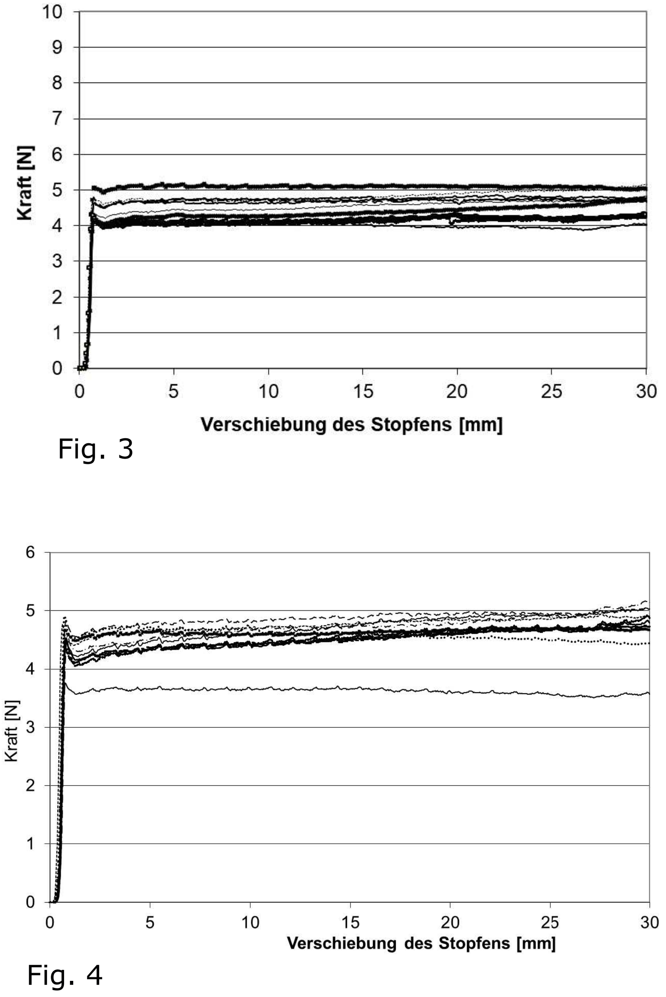

Eine mit der Zubereitung hergestellte Gleitschicht erweist sich auch als beständig gegenüber verschiedenen Behandlungen des Innenraums des Hohlkörpers. Ein Beispiel hierzu zeigt das Diagramm der Fig. 3. Wie Fig. 2 zeigt das Diagramm Messwerte der Reibkraft beim Verschieben des Stopfens auf der Gleitschicht entlang der Spritze. Es wurden wiederum zehn Exemplare vermessen. Die Spritzen wurden vor der Messung für 60 Sekunden einer Ultraschallbehandlung in Wasser unterzogen. Der Vergleich mit den Messwerten der Fig. 2 zeigt, dass die Reibung nicht beeinflusst wird, insbesondere, dass die Reibung nach der Ultraschallbehandlung nicht merklich ansteigt.

Fig. 4 zeigt analog die Messwerte der Reibkraft beim Verschieben des Stopfens auf der Gleitschicht nach einer Ultraschall-Behandlung der vermessenen Spritzen für 60 s in einer 0,1 M wässrigen NaOH-Lösung. Auch hier wird die Reibung kaum beeinflusst, was auf eine hohe Stabilität der Gleitschicht auch gegenüber hohen pH-Werten hinweist.

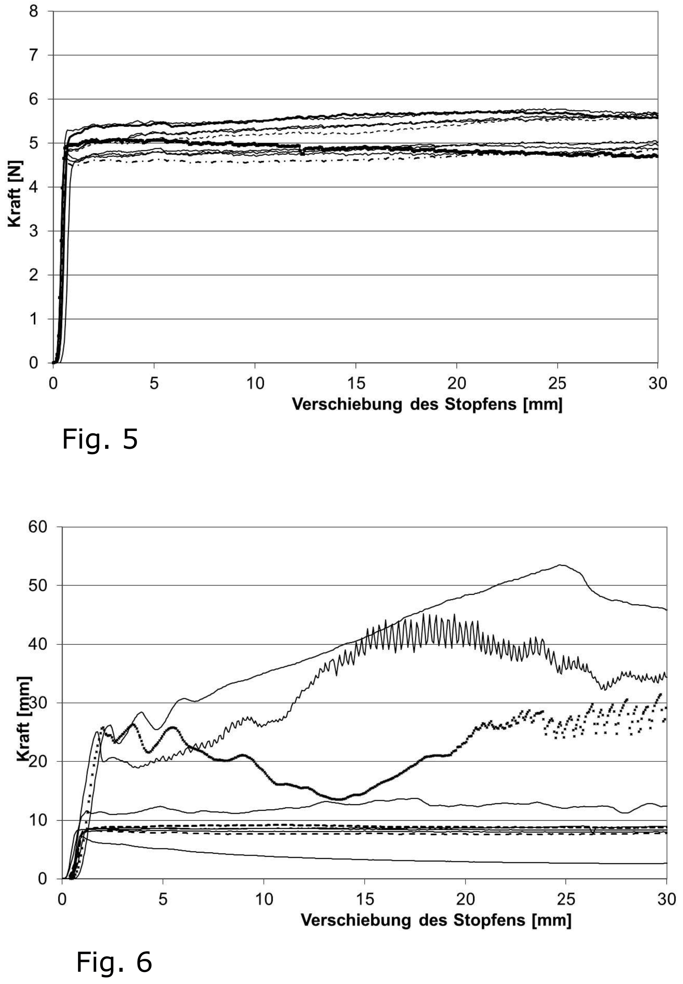

In Fig. 5 sind die Messwerte der Reibkraft beim Verschieben des Stopfens auf der Gleitschicht nach einer Ultraschallbehandlung der entsprechenden Spritze in Aceton für 60 s dargestellt. Durch die Behandlung wird die Reibung kaum beeinflusst, was für eine hohe Resistenz der Gleitschicht gegenüber polaren organischen Lösungsmitteln spricht.

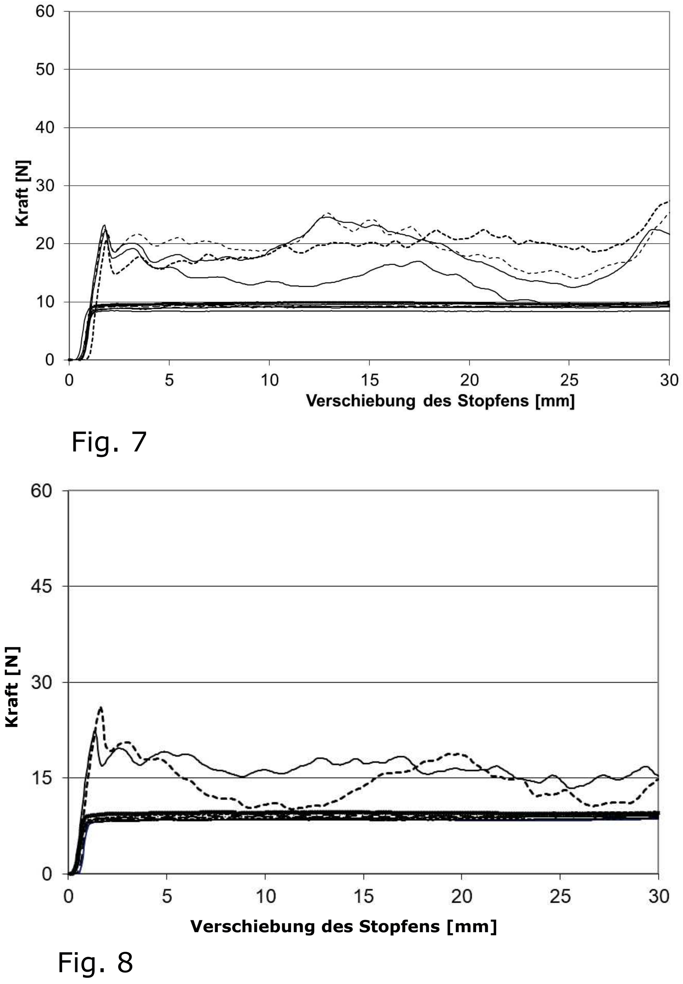

Die Figuren 6 bis 8 zeigen die Regenerationsfähigkeit der erfindungsgemäßen Gleitschichten. Die Regenerationseigenschaft wird dabei geprüft, in dem die Schichtoberfläche mit Ethylacetat (für 60 s in Ultraschall) gereinigt wird. Die HGR-Werte werden nach der Reinigung (Fig. 6) sowie ggfs. nach einer regenerativen Behandlung (z.B. Lagern, ggfs. bei erhöhter Temperatur, Fig. 7 und Fig. 8 ) vorgenommen.

Überraschender Weise hat sich gezeigt, dass der mit dem erfindungsgemäßen Verfahren hergestellte Kunststoffhohlkörper seine Haft-Gleitreibungs-Eigenschaften bei Abreinigung der Gleitschicht zumindest teilweise wieder regenerieren kann. Während eine Messung unmittelbar nach der Abreinigung (Fig. 6) deutlich erhöhte HGR-Werte zeigt, kann durch Lagerung die ursprüngliche Haft-Gleitreibung zumindest teilweise wiederhergestellt werden. Dies kann an Hand von Fig. 7 gezeigt werden. Die Spritze weist also eine Regenerationseigenschaft auf.

Aus Fig. 8 wird ersichtlich, dass leicht erhöhte Temperaturen hierzu besonders vorteilhaft sind.

Wird dagegen das Silikonnetzwerk durch mechanische Einwirkungen, beispielsweise durch Reinigung mit einem Pfeifenreiniger, weitgehend zerstört, so kann kein regenerativer Effekt mehr beobachtet werden.

### Bezugszeichenliste

| | |
|---|---|
| 1 | Packmittel |
| 3 | Spritze |
| 5 | Hohlkörper |
| 7 | zylindrischer Abschnitt |
| 10 | Gleitschicht |
| 12 | Stopfen |
| 13 | Schubstange |
| 15 | Flansch |
| 18 | Luer-Konus |

## Patentansprüche

1. Zubereitung zum Auftrag auf die Innenseite eines Hohlkörpers zur Herstellung einer Gleitschicht auf einem Packmittel für Pharmazeutika oder kosmetische Produkte, wobei die flüssige Zubereitung umfasst:
- ein reaktives Silikon-System zur Ausbildung eines Silikonnetzwerks der Gleitschicht,
- einen Katalysator zur Katalyse der Vernetzungsreaktion des reaktiven Silikon-Systems.
- zumindest ein nicht reaktives Silikonöl,
- und zumindest einen Diluenten,
wobei der Diluent eine siliziumhaltige Verbindung umfasst und wobei der Gehalt des Diluenten in der Zubereitung 45 Gewichtsprozent oder mehr und 95 Gewichtsprozent oder weniger in der Zubereitung beträgt und eine Oberflächenspannung von weniger als 19 mN/m aufweist.

2. Zubereitung gemäß dem vorstehenden Anspruch, **dadurch gekennzeichnet, dass** das reaktive Silikon-System ein Mehrkomponenten-System, vorzugsweise ein Zweikomponentensystem ist.

3. Zubereitung gemäß einem der vorstehenden Ansprüche, wobei-der Diluent eine siliziumhaltige, organische Verbindung mit höchstens 6 Siliziumatomen umfasst.

4. Zubereitung gemäß dem vorstehenden Anspruch, mit zumindest einem der folgenden Diluenten:
- Zyklisches Silikon, insbesondere Octamethylcyclotetrasiloxan, Decamethylcyclopentasiloxan,Dodecamethylcyclohexasiloxan, Tetramethylcyclotetrasiloxan, Pentamethylcyclopentasiloxan,
- Hexamethyldisiloxan (HMDSO),
- Octamethyltrisiloxan,
- Decamethyltetrasiloxan.

5. Zubereitung gemäß einem der vorstehenden Ansprüche, wobei die Zubereitung bei 23 °C eine Viskosität im Bereich von 1 bis 50000 cSt, bevorzugt 10 bis 35000 cSt, mehr bevorzugt 2500 bis 22000 cSt, besonders bevorzugt 8000 bis 10000 cSt, insbesondere 5000 bis 5500 cSt aufweist, und/oder wobei die Zubereitung bei einer Temperatur von 23°C eine Viskosität im Bereich von 0,5 bis 200 mPas, bevorzugt 1 bis 50 mPas und besonders bevorzugt im Bereich von 1 bis 10 mPas aufweist.

6. Zubereitung gemäß einem der vorstehenden Ansprüche, wobei die Zubereitung bei einer Temperatur von 23°C nach 1 s auf einer Borosilikatglasoberfläche einen Kontaktwinkel von weniger als 28°, bevorzugt weniger als 24°, besonders bevorzugt weniger als 18° und ganz besonders bevorzugt weniger als 5° aufweist.

7. Zubereitung gemäß einem der vorstehenden Ansprüche, wobei ein Tropfen der Zubereitung mit einem Volumen von 10 µl bei einer Temperatur von 23°C nach 5 s auf einer Borosilikatglasoberfläche eine Spreitung im Bereich von 7,3 und 20 mm, bevorzugt im Bereich von 7,9 und 17 mm, besonders bevorzugt im Bereich von 9,5 und 15 mm und ganz besonders bevorzugt im Bereich von 11 und 13,5 mm aufweist.

8. Verfahren zur Herstellung eines Packmittels für Pharmazeutika oder kosmetische Produkte bei welchem die Zubereitung gemäß einem der vorstehenden Ansprüche auf der Innenseite des Hohlkörpers (5) des Packmittels als Schicht aufgetragen und dann die Reaktion der Komponenten des Silikon-Mehrkomponentensystems in Gang gebracht wird, so dass sich ein Silikon-Netzwerk ausbildet und eine verfestigte Gleitschicht (10) erhalten wird.

9. Verfahren gemäß dem vorstehenden Anspruch, **dadurch gekennzeichnet, dass** die Reaktion der Komponenten des Silikon-Mehrkomponentensystems durch Erwärmung der aufgebrachten Zubereitung, bevorzugt durch Erwärmung auf eine Temperatur im Bereich von 150 bis 280 °C und/oder durch das Anlegen von Infrarotstrahlung, Konvektionsströmen oder Plasma gestartet wird.

10. Packmittel für Pharmazeutika oder kosmetische Produkte, welches einen zylindrischen Hohlkörper (5) umfasst, der innenseitig mit einer Gleitschicht (10) beschichtet ist, wobei die Gleitschicht (10) ein Silikon-Netzwerk aufweist, in welchem ein Silikonöl aufgenommen ist, wobei der Hohlkörper (5) derart ausgestaltet ist, dass ein Stopfen in den Hohlkörper (5) eingeführt werden kann und der Haftreibungswert der Reibung eines in den Hohlkörper (5) eingesetzten Stopfens an der Gleitschicht (10) maximal 20% größer, als der mittlere Gleitreibungswert ist und wobei die Schichtdicke der Gleitschicht (10) weniger als 3 µm beträgt.

11. Packmittel gemäß dem vorstehenden Anspruch, wobei der Haftreibungswert der Reibung eines in den Hohlkörper (5) eingesetzten Stopfens an der Gleitschicht (10) maximal 10% größer, bevorzugt maximal 5% größer als der mittlere Gleitreibungswert ist und/oder wobei das Verhältnis µ_{G}/µ_{H} von Gleitreibungskoeffizient µ_{G} und Haftreibungskoeffizient µ_{H} größer als 0,80, bevorzugt größer als 0,9 und besonders bevorzugt größer als 0,95 ist.

12. Packmittel gemäß einem der vorstehenden Ansprüche 10 oder 11, wobei bei Bewegung eines Standardstopfens mit einer Geschwindigkeit von 100 mm/min über die Gleitschicht (10) der Haftreibungswert und/oder der Gleitreibungswert der Reibung des in den Hohlkörper (5) eingesetzten Stopfens kleiner 10 N, bevorzugt kleiner 8 N und besonders bevorzugt kleiner 6 N ist

13. Packmittel gemäß einem der vorstehenden Ansprüche 10 bis 12, wobei sich nach einer Entfernung des Silikonöls von der Oberfläche der Gleitschicht (10) die Gleitwirkung der Gleitschicht (10) regeneriert und/oder die Schichtdicke der Gleitschicht (10) t weniger als **1** µm, sowie mindestens 0,4 µm, bevorzugt mindestens 0,5 µm beträgt .

14. Packmittel gemäß einem der vorstehenden Ansprüche 10 bis 13, wobei die Gleitschicht (10) unmittelbar auf der Innenseite des Hohlkörpers (5) aufgebracht ist.

15. Packmittel gemäß einem der vorstehenden Ansprüche 10 bis 14, **dadurch gekennzeichnet, dass** das Packmittel eine Spritze oder Karpule ist und die Gleitschicht (10) vorzugsweise zumindest den Bereich der Innenseite des Hohlkörpers (5) bedeckt, über den der Stopfen der Spritze oder Karpule gleiten kann.
